(19) Europäisches Patentamt
European Patent Office
Office européen des brevets

(11) Veröffentlichungsnummer: **0 250 360**
**A2**

(12) **EUROPÄISCHE PATENTANMELDUNG**

(21) Anmeldenummer: **87810321.7**

(22) Anmeldetag: **03.06.87**

(51) Int. Cl.4: **C 07 C 43/29**, C 07 C 149/34, C 07 C 41/00, A 01 N 31/14

(30) Priorität: **10.06.86 CH 2338/86**

(43) Veröffentlichungstag der Anmeldung: **23.12.87 Patentblatt 87/52**

(84) Benannte Vertragsstaaten: **BE CH DE ES FR GB IT LI NL**

(71) Anmelder: **CIBA-GEIGY AG, Klybeckstrasse 141, CH-4002 Basel (CH)**

(72) Erfinder: **Gsell, Laurenz, Dr., Maiengasse 56, CH-4056 Basel (CH)**
Erfinder: **Ackermann, Peter, Dr., Hangelimattweg 113, CH-4148 Pfeffingen (CH)**

(54) **Substituierte Benzyl-cyclopropyl-methyl-äther.**

(57) Neue gegebenenfalls substituierte Phenoxyfluorbenzyl-tetramethyl-cyclopropylmethyl-äther der Formel

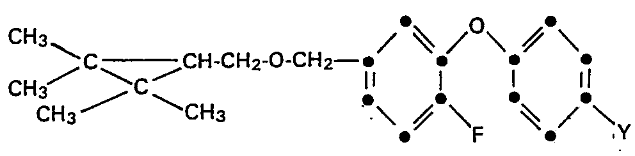

(I)

worin

Y Wasserstoff, Halogen, $C_1$-$C_4$-Alkyl, mit 1 bis 9 Halogenatomen substituiertes $C_1$-$C_4$-Alkyl, $C_1$-$C_4$-Alkoxy, mit 1 bis 9 Halogenatomen substituiertes $C_1$-$C_4$-Alkoxy, $C_1$-$C_4$-Alkylthio, mit 1 bis 9 Halogenatomen substituiertes $C_1$-$C_4$-Alkylthio, $C_2$-$C_4$-Alkenyl oder $C_2$-$C_4$-Alkinyl bedeutet.

Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten, speziell deren larvale Stadien. Die neuen Verbindungen weisen vor allem gute Wirksamkeit gegen pflanzenschädigende Insekten in Reiskulturen auf.

EP 0 250 360 A2

CIBA-GEIGY AG  5-15928/+

Basel (Schweiz)

Substituierte Benzyl-cyclopropylmethyl-äther

Die vorliegende Erfindung betrifft neuartige, gegebenenfalls substituierte (3-Phenoxy-4-fluorbenzyl)-(2,2,3,3-tetramethylcyclopropylmethyl)-äther, Verfahren und Zwischenprodukte zu ihrer Herstellung sowie ihre Verwendung in der Schädlingsbekämpfung.

Die neuen erfindungsgemässen Aether haben die Formel I

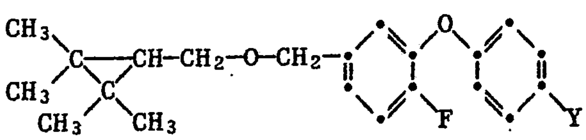

(I)

worin

Y Wasserstoff, Halogen, $C_1$-$C_4$-Alkyl, mit 1 bis 9 Halogenatomen substituiertes $C_1$-$C_4$-Alkyl, $C_1$-$C_4$-Alkoxy, mit 1 bis 9 Halogenatomen substituiertes $C_1$-$C_4$-Alkoxy, $C_1$-$C_4$-Alkylthio, mit 1 bis 9 Halogenatomen substituiertes $C_1$-$C_4$-Alkylthio, $C_2$-$C_4$-Alkenyl oder $C_2$-$C_4$-Alkinyl bedeutet.

Im Rahmen der vorliegenden Erfindung ist unter Halogen Fluor, Chlor, Brom oder Jod, insbesondere aber Fluor oder Chlor, zu verstehen.

Alkyl-, Halogenalkyl-, Alkoxy-, Halogenalkoxy-, Alkylthio- und Halogenalkylthiogruppen Y können geradkettig oder verzweigt sein. Beispiele solcher Gruppen sind u.a.: Methyl, Trifluormethyl, Methoxy, Difluormethoxy, Aethyl, Aethoxy, Propyl, Isopropyl, n-Butyl, i-Butyl usw.; die bevorzugten Alkenylgruppen sind Vinyl, Propenyl und Allyl.

Hervorzuheben wegen ihrer biologischen Wirkung sind Verbindungen der Formel I, worin

Y Wasserstoff, Halogen, $C_1$-$C_3$-Alkyl, Methoxy, Aethoxy, Methylthio Trifluormethyl, Vinyl, Propargyl oder Aethinyl bedeutet.

Insbesondere bevorzugt werden solche Verbindungen der Formel I, worin

Y Wasserstoff, Fluor, Brom, Chlor, Jod, Methyl, Methoxy, Vinyl und Aethinyl bedeutet und solche, worin

Y Wasserstoff, Fluor, Methyl oder Methoxy bedeutet.

Die Verbindungen der Formel I werden in an sich bekannter Weise hergestellt, indem man

a) die Verbindung der Formel II

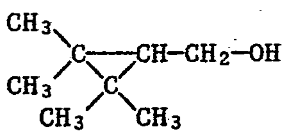

(II)

mit einer Verbindung der Formel III

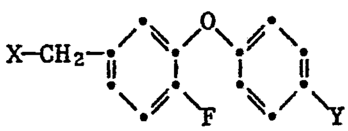

(III)

oder

b) eine Verbindung der Formel IV

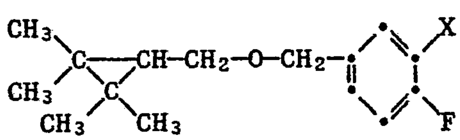

(IV)

mit einer Verbindung der Formel V

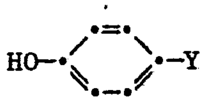

(V)

umsetzt, wobei Y die vorstehend angegebenen Bedeutungen besitzt und X für ein Halogenatom, vorzugsweise Jod oder Brom steht.

Die obigen Verfahren werden bei einer Reaktionstemperatur zwischen -10 und 120°C, meist zwischen 20 und 80°C, bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoff, insbesondere Benzol, Toluol, Xylole, Chloroform und Chlorbenzol; Nitrile, wie Acetonitril; Dimethylsulfoxid und Ketone wie Aceton und Methylketon sowie Hexan. Die bei Verfahren a) stattfindende Verätherung wird mit Vorteil in Gegenwart von Basen, wie Alkalihydroxyden oder -carbonaten, speziell aber Metallhydriden, z.B. Natriumhydrid, durchgeführt. Die bei Verfahren b) stattfindende Verätherung zum Biphenyläther wird entweder unter den üblichen Bedingungen der Ullmann-Kondensation durchgeführt oder entsprechend dem Verfahren a).

Die Ausgangsstoffe der Formeln II, III und V sind bekannt und können analog bekannter Methoden hergestellt werden. So wird die Herstellung des Tetramethylcyclopropylmethanols der Formel II in "Tetrahedron Letters" 34, 3331-35, beschrieben. Die Phenoxybenzylderivate vom Typ der Formel III und deren Herstellung sind z.B. bekannt aus der EP-Patentanmeldung 0,125,204, der GB-Patentanmeldung 2,085,006 und der DE-OS 2709355. Die Benzyläther der Formel IV sind neu und bilden ebenfalls einen Gegenstand der vorliegenden Erfindung; sie können hergestellt werden durch Umsetzung des Tetramethylcyclopropylmethanols der Formel II mit einem entsprechend substituierten Benzylderivat der Formel VI

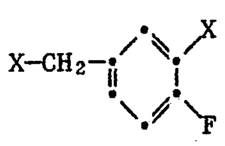

(VI),

worin X die vorstehend angegebene Bedeutung hat.

Es wurde gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tieren befallenden Schädlingen. In diesem Zusammenhang wird auf die sehr geringe Fisch-Toxizität der erfindungsgemässen Verbindungen hingewiesen, ein für die Anwendung in Reis-Kulturen wichtiger Aspekt.

Insbesondere eignen sich die Verbindungen der Formeln I bzw. Ia) zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Neben ihrer Wirkung gegenüber Mücken und Fliegen, wie z.B. Aëdes aegypti und Musca domestica, können Verbindungen der Formel I auch zur Bekämpfung von pflanzenschädigenden Frassinsekten in Zier- und Nutzpflanzungen, insbesondere in Baumwollkulturen (z.B. gegen Spodoptera littoralis und Heliothis virescens) sowie in Getreide-, Obst- und Gemüsekulturen (z.B. gegen Laspeyresia pomonella, Leptinotarsa decemlineata und Epilachna varivestis) eingesetzt werden. Die Verbindungen der Formel I zeichnen sich auch durch gute Wirkung gegen larvale Insektenstadien und Nymphen, insbesondere von fressenden Schadinsekten, aus. Auch können die Verbindungen der Formel I mit ausgezeichnetem Erfolg gegen pflanzenschädigende Zikaden, speziell in Reiskulturen, eingesetzt werden.

Die Verbindungen eignen sich ferner zur Bekämpfung von Ektoparasiten, z.B. Lucilia sericata, sowie von Zecken an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie

N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag
München/Wien 1981.

Die pestiziden Zubereitungen enthalten - auf das Gewicht bezogen -in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung des (3-Phenoxy-4-fluorbenzyl)-(2,2,3,3-tetramethylcyclopropylmethyl)-äthers

Es werden 1,9 g 2,2,3,3-Tetramethylcyclopropylmethanol und 4,2 g 3-Phenoxy-4-fluorbenzylbromid, gelöst in 20 ml eines Gemisches aus Toluol und Dimethylformamid (1/1) bei 0-5°C zu 0,9 g Natriumhydrid (50%ige Dispersion in Mineralöl) in 60 ml Toluol/Dimethylformamid (1/1) tropfenweise hinzugefügt. Nach Abklingen der Reaktion wird der Ansatz während 16 Stunden bei Raumtemperatur ausgerührt, gesättigte Ammoniumchlorid-Lösung zugetropft und mit Toluol extrahiert. Die vereinigten Toluol-Extrakte werden mit gesättigter Kochsalzlösung gewaschen, über $MgSO_4$ getrocknet und am Rotationsverdampfer eingeengt. Das erhaltene Rohprodukt wird an 300 g Kieselgel mit Hexan-Aether (95/5) chromatographiert. Die Titelverbindung der Formel

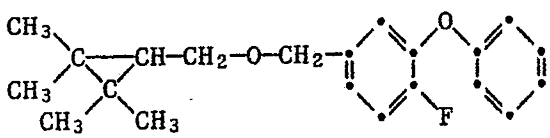

wird als klares Oel vom Brechungsindex $n_D^{24}$ = 1,5300 (Verbindung Nr. 1) erhalten.

Wie vorstehend beschrieben, sind auch die folgenden Verbindungen der Formel I erhältlich:

| Verbindung Nr. | Y | physik. Daten |
|---|---|---|
| 2 | F | $n_D^{25}$ = 1,5186 |
| 3 | $-CH_3$ | $n_D^{25}$ = 1,5293 |
| 4 | -Cl | $n_D^{25}$ = 1,5343 |

Beispiel 2: Herstellung des (3-Brom-4-fluorbenzyl)-(2,2,3,3-tetramethylcyclopropylmethyl)-äthers (Ausgangsverbindung gemäss Formel IV)

Es werden 20 g 2,2,3,3-Tetramethylcyclopropylmethanol und 41,9 g 3-Brom-4-fluorbenzylbromid gelöst in 100 ml eines Gemisches aus Toluol und Dimethylformamid (1/1) bei 0-10°C zu 8,2 g Natriumhydrid (50%ige Dispersion in Mineralöl) in 200 ml Toluol/Dimethylformamid (1/1) tropfenweise zugegeben. Nach Abklingen der Reaktion wird der Ansatz für weitere 16 Stunden ausgerührt, gesättigte Ammoniumchlorid-Lösung zugetropft und mit Toluol extrahiert. Die Toluol-Extrakte werden mit gesättigter Kochsalzlösung gewaschen, über $MgSO_4$ getrocknet und am Rotationsverdampfer vom Lösungsmittel befreit. Das erhaltene Rohprodukt wird an Kieselgel und mit einem Hexan/Toluol-Gemisch (5/1) chromatographisch gereinigt. Man erhält die Titelverbindung als klares Oel vom Brechungsindex $n_D^{24}$ = 1,5128.

Beispiel 3: Formulierungen für Wirkstoffe der Formel I gemäss Beispiel 1 oder Kombinationen dieser Wirkstoffe mit anderen Insektiziden oder Akariziden (% = Gewichtsprozent):

| 3.1. Emulsions-Konzentrate | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| | a) | b) | c) |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 3.2. Lösungen | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3.3. Granulate | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 3.4. Stäubemittel | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Beispiel 4: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 5: Wirkung gegen Aedes aegypti (Larven)

Auf die Oberfläche von 150 ml Wasser, das sich in einem Behälter befindet, wird so viel einer 0,1%igen acetonischen Lösung des Wirkstoffes pipettiert, dass eine Konzentration von 200 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen der Formel I gemäss Beispiel 1 zeigen gute Wirkung im obigen Test.

Beispiel 6: Frassgift-Wirkung auf Laodelphax striatellus und Nilaparvata lugens (Nymphen);
Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I gemäss Beispiel 1 zeigen gute Wirkung in diesem Test.

Beispiel 7: Ovizide Wirkung auf Laodelphax striatellus und Nilaparvata lugens;
Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedelung schlüpfen die jungen Zikaden, und es erfolgt die Auszählung. Aus dem Vergleich der Anzahl geschlüpfter Larven auf den behandelten Pflanzen zu den auf unbehandelten Kontrollpflanzen geschlüpften Tieren wird die prozentuale Mortalität bestimmt.

Verbindungen der Formel I gemäss Beispiel 1 zeigen in obigem Test gute ovizide Wirkung.

Beispiel 8: Insektizide Frassgift-Wirkung

Ca. 25 cm hohe eingetopfte Baumwollpflanzen werden mit wässrigen Wirkstoffemulsionen besprüht, die den Wirkstoff in einer Konzentration von 400 ppm enthalten.

Nach dem Antrocknen des Sprühbelages werden die Baumwollpflanzen mit Spodoptera littoralis- bzw. Heliothis virescens-Larven im ersten larvalen Stadium besiedelt. Der Versuch wird bei 24°C und etwa 60 % relativer Luftfeuchtigkeit durchgeführt. Nach 120 Stunden wird die %-Mortalität der Test-Insekten gegenüber unbehandelten Kontrollen bestimmt.

Die Verbindung Nr. 1 gemäss Beispiel 1 zeigt 80-100 % Wirkung (Mortalität) gegen Spodoptera-Larven in diesem Test.

Beispiel 9: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für

5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Die erfindungsgemässen Verbindungen gemäss Beispiel 1 zeigen in diesem Test gute Wirkung.

Beispiel 10: Wirkung gegen Bemisia tabaci

Eingetopfte Baumwollpflanzen im Keimblattstadium werden mit Bemisia tabaci (weisse Fliegen) in der Art besiedelt, dass 40 ungesexte Adulte pro Pflanze angesetzt werden. Nach erfolgter Eiablage während drei Tagen werden alle vorhandenen Adulten entfernt. Zehn Tage nach der Besiedlung, d.h. zu einem Zeitpunkt, wo sich etwa 2/3 der Nymphen im ersten Nymphenstadium und 1/3 im zweiten Stadium befinden, werden die besiedelten Pflanzen bis zur Tropfnässe mit einer wässrigen Zubereitung des zu prüfenden Wirkstoffes (Konzentration 400 ppm) besprüht. Die Auswertung erfolgt 24 Tage nach der Besiedlung auf abgetötete und lebende Nymphen, Puppen und Adulte. Der Versuch wird in einer Gewächshauskabine bei 25°C und einer relativen Luftfeuchtigkeit von etwa 50 % bis 60 % durchgeführt.

Die Verbindungen der Formel I gemäss dem vorliegenden Beispiel 1 zeigen gute Wirkung in diesem Test.

Patentansprüche

1. Verbindung der Formel 1

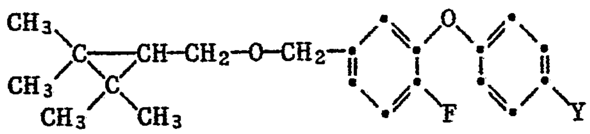

(I)

worin

Y Wasserstoff, Halogen, $C_1$-$C_4$-Alkyl, mit 1 bis 9 Halogenatomen substituiertes $C_1$-$C_4$-Alkyl, $C_1$-$C_4$-Alkoxy, mit 1 bis 9 Halogenatomen substituiertes $C_1$-$C_4$-Alkoxy, $C_1$-$C_4$-Alkylthio, mit 1 bis 9 Halogenatomen substituiertes $C_1$-$C_4$-Alkylthio, $C_2$-$C_4$-Alkenyl oder $C_2$-$C_4$-Alkinyl bedeutet.

2. Verbindung gemäss Anspruch 1 der Formel I, dadurch gekennzeichnet, dass

Y Wasserstoff, Halogen, $C_1$-$C_3$-Alkyl, Methoxy, Aethoxy, Methylthio Trifluormethyl, Vinyl, Propargyl oder Aethinyl bedeutet.

3. Verbindung gemäss Anspruch 2 der Formel 1, dadurch gekennzeichnet, dass

Y Wasserstoff, Fluor, Brom, Chlor, Jod, Methyl, Methoxy, Vinyl oder Aethinyl bedeutet.

4. Verbindung gemäss Anspruch 3, der Formel 1, dadurch gekennzeichnet, dass

Y Wasserstoff, Halogen oder Methyl bedeutet.

5. Verbindung gemäss Anspruch 4 der Formel

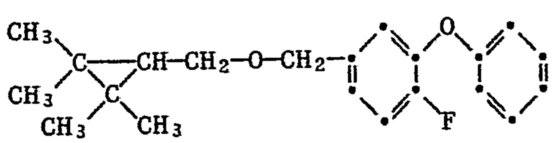

6. Verfahren zur Herstellung einer Verbindung der Formel I gemäss Anspruch 1, dadurch gekennzeichnet, dass man

a) die Verbindung der Formel II

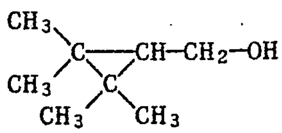

(II)

mit einer Verbindung der Formel III

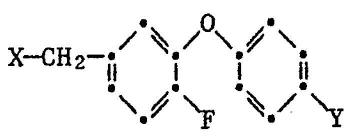

(III)

oder

b) eine Verbindung der Formel IV

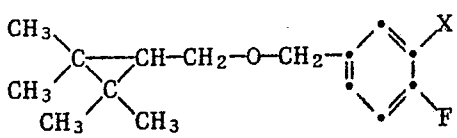

(IV)

mit einer Verbindung der Formel V

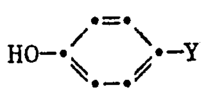

umsetzt, wobie Y die in Anspruch 1 angegebenen Bedeutungen besitzt und X für ein Halogenatom, vorzugsweise Brom oder Jod, oder die p-Toluolsulfonatgruppe steht.

7. Verbindung der Formel IV gemäss Anspruch 6, worin X die in Anspruch 1 angegebenen Bedeutungen besitzt.

8. Schädlingsbekämpfungsmittel, welches als aktive Komponente eine Verbindung gemäss einem der Ansprüche 1 bis 5 zusammen mit geeigneten Trägern und/oder anderen Zuschlagstoffen enthält.

9. Verwendung einer Verbindung gemäss einem der Ansprüche 1 bis 5 zur Bekämpfung von Insekten und Vertretern der Ordnung Akarina an Tieren und Pflanzen.

10. Verwendung gemäss Anspruch 9 zur Bekämpfung von pflanzenschädigenden Insekten.

11. Verwendung gemäss Anspruch 10 zur Bekämpfung von pflanzenschädigenden Insekten in Reiskulturen.

12. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der Ansprüche 1 bis 5 oder mit einem Mittel enthaltend neben Zusatz- und Trägerstoffen eine pestizid wirksame Menge dieser Verbindung, in Kontakt bringt oder behandelt.

FO 7.5/EIC/ga*/cp*